# EUROPÄISCHE PATENTSCHRIFT

(11) **EP 1 651 767 B1**
(45) Veröffentlichungstag und Bekanntmachung des Hinweises auf die Patenterteilung: **06.11.2013**
(21) Anmeldenummer: 04763579.2
(22) Anmeldetag: 27.07.2004
(51) Int. Cl.: C12N 15/67, C12P 21/02, C12N 1/21, C12R 1/19

(54) **VERWENDUNG EINES LYSATES ZUR ZELLFREIEN PROTEINBIOSYNTHESE**
USE OF A LYSATE FOR CELL-FREE PROTEIN BIOSYNTHESIS
UTILISATION D'UN LYSAT POUR LA BIOSYNTHESE DE PROTEINES IN VITRO

(30) Priorität: 06.08.2003 DE 10336705
(43) Veröffentlichungstag der Anmeldung: 03.05.2006
(73) Patentinhaber: QIAGEN GmbH, 40724 Hilden (DE)
(72) Erfinder: GERRITS, Michael, 14195 Berlin (DE); STREY, Jan, 14195 Berlin (DE); STIEGE, Wolfgang, 14195 Berlin (DE)
(74) Vertreter: Jungblut, Bernhard Jakob
(86) Internationale Anmeldenummer: PCT/EP2004/008469
(87) Internationale Veröffentlichungsnummer: WO 2005/017166

(56) Entgegenhaltungen:
- WO-A-90/05785
- WO-A-02/053582
- SHIMIZU Y ET AL: "CELL-FREE TRANSLATION RECONSTITUTED WITH PURIFIED COMPONENTS" NATURE BIOTECHNOLOGY, NATURE PUBLISHING, US, Bd. 19, August 2001 (2001-08), Seiten 751-755, XP001060378 ISSN: 1087-0156 in der Anmeldung erwähnt
- SHORT GLENN F III ET AL: "Effects of release factor 1 on in vitro protein translation and the elaboration of proteins containing unnatural amino acids" BIOCHEMISTRY, AMERICAN CHEMICAL SOCIETY, EASTON, PA, US, Bd. 38, Nr. 27, 6. Juli 1999 (1999-07-06), Seiten 8808-8819, XP002195367 ISSN: 0006-2960 in der Anmeldung erwähnt
- LAMLA T ET AL: "The cell-free protein biosynthesis--applications and analysis of the system." ACTA BIOCHIMICA POLONICA. 2001, Bd. 48, Nr. 2, 2001, Seiten 453-465, XP002307274 ISSN: 0001-527X
- ITO K. ET AL: "Single amino acid substitution in prokaryote polypeptide release factor 2 permits it to terminate translation at all three stop codons", PROC. NATL. ACAD. SCI, vol. 95 , pages 8165-8169, USA

## Beschreibung

### Gebiet der Erfindung

Die Erfindung betrifft ein Verfahren zur zellfreien Proteinbiosynthese von synthetischen Proteinen umfassend ein Verfahren zur Herstellung eines Lysates, wobei das Lysat eine geringe Aktivität essentieller Translationsprodukte aufweist.

### Hintergrund der Erfindung

Proteine werden für biotechnologische und medizinische Anwendungen in hoher Reinheit, insbesondere aber auch in hoher Menge benötigt. Meist ist eine klassische Synthese kaum möglich, jedenfalls in der Regel unwirtschaftlich. Dies betrifft besonders die Herstellung modifizierter Proteine bzw. von Proteinen, die nicht-natürliche Aminosäuren enthalten.

Eine Möglichkeit der Herstellung von Proteinen in größerem Maßstab ist die gentechnische Herstellung. Hierzu wird klonierte DNA, welche für das gewünschte Protein codiert, als fremde DNA in Form von Vektoren bzw. Plasmiden in Zellen, insbesondere prokaryontische Zellen, eingeschleust. Diese Zellen werden dann kultiviert, wobei die durch die fremde DNA codierten Proteine exprimiert und gewonnen werden. Zwar lassen sich auf diesem Wege bereits gesteigerte Mengen an Protein gewinnen, die insofern bekannten Maßnahmen, insbesondere die Klonierung, sind jedoch aufwendig.

Zudem sind die Zellen meist nur transient transfiziert und nur in Ausnahmefällen stabil immortalisiert. Desweiteren beherbergt die in vivo Proteinbiosynthese mehrere Nachteile: Das zelleigene Expressionssystem unterdrückt die Expression heterologer Genstrukturen oder entsprechende mRNA oder Genprodukte sind instabil oder werden durch intrazelluläre Nukleasen bzw. Proteasen zerstört. Bei toxischen Endprodukten führt die Expression zur Hemmung oder gar zum Absterben des Organismus. Diese Probleme führen dazu, dass eine deutliche Überproduktion des erwünschten Proteins kaum möglich ist.

Die zellfreie Proteinbiosynthese stellt eine effektive Alternative zur Synthese von Proteinen durch genetisch veränderte Organismen dar, da hier die obengenannten Phänomene vermeidbar sind. Bekannte zellfreie Proteinbiosynthesesysteme sind Lysate von Kaninchenreticulocyten, aus Weizenkeimlingen und bakteriellen S30-Extrakten. Methoden zur Herstellung eines Lysates sind dem Fachmann wohl bekannt. Jedoch weiterhin problematisch bei der Verwendung eines Lysates ist, dass im Lysat Komponenten enthalten sein können, welche störend in die Produktion des gewünschten Proteins eingreifen und somit die Ausbeute reduzieren. Der negative Effekt solcher Komponenten kann durch deren Inhibierung oder Entfernung aus dem Lysat unterbunden werden. Störende Aktivitäten werden bei der Herstellung von Lysaten allein dadurch entfernt, dass der Inhalt der Zelle im Verlauf der Aufarbeitung der für die Proteinbiosynthese wichtigen Komponenten fraktioniert wird. Während dieses Prozesses werden beispielsweise Membran- und Zellwandkomponenten, ein großer Teil der chromosomalen DNA sowie niedermolekulare Komponenten abgetrennt. Verbliebene Aktivitäten müssen in weiteren Aufarbeitungsschritten beseitigt oder vorab durch genetische Modifizierung des Organismus verhindert werden.

Aus der Literaturstelle US 6,337,191 ist die Verwendung eines Lysates zur Herstellung von Proteinen mit einem verbesserten Energieregenerierungssystem, in welchem optional störende Enzymaktivitäten durch Inhibierung oder Entfernung der unerwünschten Enzyme zusätzlich eleminiert werden, bekannt. Mögliche Methoden sind das Knockoutverfahren, Antisense- oder weitere bekannte Verfahren zur Entfernung von Proteinen, wie Affinitätschromatographien.

Auch sind Lysate aus gentechnisch veränderten Zellstämmen bekannt, welche defizient an bestimmten Aktivitäten sind. Beispielhaft sei hier der genetisch veränderte E. coli-Stamm EcoPro T7 der Firma Novagen genannt, welcher defizient an den Proteasen lon und ompT ist.

In besonderen Fällen ist das die in vitro Proteinbiosynthese störende Protein für das Wachstum des Organismus unerläßlich. Eine Inaktivierung des Enzyms führt zwangsläufig zum Absterben des Organismus. In solchen Fällen ist das Enzym nachträglich zu inaktivieren oder zu entfernen. Die bereit genannte Literaturstelle US 6,337,191 gibt hierzu diverse Methoden an.

Im Wege der zellfreien Proteinbiosynthese lassen sich insbesondere synthetische Proteine, welche unnatürliche Aminosäuren enthalten, herstellen. Hierbei wird das Codon einer Aminosäure durch Mutation in ein Nonsense-Codon entsprechend einem Terminationscodon umgewandelt. Der Einbau unnatürlicher Aminosäuren erfolgt durch zu diesem Terminationscodon komplementäre tRNAs, welche mit den unnatürlichen Aminosäuren synthetisch beladen sind. Das Terminationscodon UAG steht für das Amber-Codon, entsprechend werden die dem Terminationscodon UAG komplementäre tRNAs als amber-Suppressor-tRNAs bezeichnet. Der Einbau von unnatürlichen Aminosäuren mit Hilfe von Amber-Suppressor-tRNAs am UAG Stop-Codon steht jedoch in direkter Konkurrenz zum Abbruch der Kettenbildung durch den natürlichen Terminationsfaktor 1 (RF1). Unter Umständen ist die Konkurrenz so stark, dass nur ein sehr geringer Teil der Aminoacyl-tRNA für die Proteinsynthese genutzt wird und ein unerwünscht großer Anteil der Kapazität des Translationssystems für die Synthese terminierter Peptide genutzt wird. Folge dieses Konkurrenzverhaltens ist ein schlechter Einbau der unnatürlichen Aminosäure und somit eine niedrige Ausbeute an modifiziertem Protein, verbunden mit einer hohen Anzahl von unerwünschten Nebenprodukten bestehend aus vorzeitig abgebrochenen oder terminierten Proteinketten.

In der Literaturstelle Shimizu et al.; Nature Biotech 19 (8): 751 - 755, 1991 ist ein Pure-System beschrieben, in welchem eine Suppressor-tRNA effizient funktioniert, wenn RF 1 weggelassen wird.

Aus der Literaturstelle Short et al.; Biochemistry 38: 8808 - 8819, 1999 ist ein Temperatur sensitiver Terminationsfaktor 1 aus E. Coli bekannt, welcher durch mildes Erhitzen des Lysates inaktiviert wird. Die Steigerung der Ausbeute mit unnatürlichen Aminosäuren modifizierter Proteine ist signifikant. Ebenso sind weniger Nebenprodukte bei der Herstellung des Proteins DHFR zu verzeichnen. Nachteilig an diesem Verfahren ist die Erwärmung des Lysates, durch welche weitere thermosensitive Faktoren des Proteinbiosyntheseapparates zerstört werden.

Das Dokument WO02/053582A (POST GENOME INST CO LTD) offenbart eine Methode zur Entfernung von Therminationsfaktoren durch gentechnische Markierung und Abtrennung aus einem Translationssystem zur zellfreien Proteinbiosynthese. Hierbei ist das Translationssystem synthetisch und es werden alle Faktoren des Translationssystems markiert, wobei zumindest bei einigen Faktoren eine Verringerung der Aktivität durch die Markierung erwartet werden kann.

### Technisches Problem der Erfindung

Der Erfindung liegt das technische Problem zugrunde, ein alternatives Verfahren zur Proteinbiosynthese anzugeben, welches ein Zelllysat verwendet, welches eine verminderte Menge oder Aktivität an Terminationsfaktor aufweist.

### Definitionen

Der Begriff "Lysat" umfaßt alle durch Aufschluß eukaryontischer oder prokaryontischer Zellen hergestellte aktiven Zellextrakte.

Unter "essentiellen Translationsprodukten" werden Genprodukte verstanden, welche für das Überleben und/oder die Vermehrung einer Zelle zwingend notwendig sind.

Unter "synthetischen Proteinen" werden zellfrei hergestellte Proteine verstanden.

"Reduzierte Ausbeute" bezeichnet, dass die Ausbeute eines synthetischen Proteins durch zellfreie Proteinbiosynthese in einem Lysat, welches das essentielle Translationsprodukt enthält, 10% bezogen auf Gewichte, vorzugsweise 20 % bis 80%, besonders bevorzugt über 90%, geringer ist als die Ausbeute des gleichen synthetischen Proteins in einem Lysat gleicher Art und bei ansonsten gleichen Bedingungen, aus welchem jedoch das essentielle Translationsprodukt abgetrennt wurde.

Eine "Markersequenz" stellt eine Struktur dar, welche der Identifizierung von Molekülen u.a. Proteinen dient. Eine solche Struktur kann eine kurze Sequenz von Aminosäuren sein, wobei die Anzahl an Aminosäuren bevorzugt kleiner 10, insbesondere zwischen 4 bis 8 ist. Beispielhaft ist eine solche Struktur ein Tag. Eine Markersequenz kann auch für Enzyme codieren, anhand derer das markierte Molekül identifiziert und auch abgetrennt werden kann.

Eine "Selektionssequenz" codiert für eine Struktur, der unter bestimmten selektiven Bedingungen nur dem Träger dieser Selektionssequenz ein überleben erlauben. In der Regel handelt es sich um Resistenzgene gegenüber bestimmten Antibiotika. Weitere Selektionssequenzen können aus dem Stoffwechsel der Nukleinsäuren oder der Aminosäuren stammen.

Der Begriff der "Lyse" bezeichnet die Auflösung von Zellen durch Zerstörung der Zellwand bzw. Zellmembran entweder unter Mitwirkung lytischer Enzyme oder durch mechanische oder chemische Einwirkung.

### Grundzüge der Erfindung

Zur Lösung des technischen Problems lehrt die Erfindung ein Verfahren zur zellfreien Proteinbiosynthese umfassend ein Verfahren zur Herstellung eines Zelllysates mit folgenden Verfahrensschritten:
a) Austausch einer für ein essentielles, jedoch die Ausbeute einer zellfreien Proteinbiosynthese reduzierendes Translationsprodukt, ausgewählt aus Terminationsfaktor 1 (RF1), Terminationsfaktor 2 (RF2) und eukaryontischer Terminationsfaktor (eRF), codierenden genomischen Sequenz in einem Organismus gegen eine unter einem geeigneten regulatorischen Element stehende fremde DNA, wobei die fremde DNA für das essentielle Translationsprodukt, jedoch zusätzlich enthaltend eine Markersequenz co-diert,
b) Kultivieren des gemäß a) transformierten Organismus,
c) Lyse des Organismus aus der Kultur nach b) und
d) Abtrennung des essentiellen Translationsproduktes aus dem in c) erhalte-nen Lysat mittels eines für die Markersequenz selektiven Trennverfahrens.

Das regulatorische Element kann ebenfalls fremd sein, es kann sich aber auch um ein natürlicherweise vorliegendes regulatorisches Element handeln. In ersterem Falle muß auch das regulatorische Element, im gleichen Verfahrensschritt, wie die Einführung der fremden DNA oder in einem hiervon verschiedenen Verfahrensschritt, eingeführt werden.

Die Herstellung des erfindungsgemäßen Lysates ist einfach, und das erhaltene Lysat erlaubt erhöhte Ausbeuten an synthetischem Protein in zellfreien Proteinbiosynthese-Verfahren, insbesondere eine hohe Ausbeute an Proteinen mit nicht-natürlichen Aminosäuren.

Dies wird dadurch erreicht, dass das essentielle Translationsprodukt mit einer Markersequenz versehen wird, durch welche das essentielle Translationsprodukt über die Affinität der Markersequenz aus dem Lysat entfernt oder seine Aktivität inhibiert wird. Die Modifizierung des essentiellen Translationsproduktes erfolgt im chromosomalen Gen des Proteins, so daß das essentielle Translationsprodukt mit der Markersequenz fusioniert exprimiert wird. Eine Markersequenz codiert für eine Struktur, welche eine hohe Affinität für (meist immobilisierte) Bindungsstellen in Trennsystemen zur Aufreinigung oder zu Inhibitoren aufweist. Dadurch kann die Aktivität des Terminationsfaktors aus einer Mixtur von Proteinen oder einer Mixtur von beliebigen Molekülen, welche die Markersequenz nicht enthalten, entfernt werden.

Durch den Einbau der Markersequenz in das chromosomale Gen des Terminationsfaktors des Organismus wird eine stabile Transformation des Organismus erreicht. Unter eine stabile Transformation des Organismus erreicht. Unter dieser Voraussetzung ist eine Kultivierung des gentechnisch veränderten Organismus ohne Verlust seiner zusätzlichen genetischen Information möglich, und zwar auch ohne Selektionsdruck.

Ein bevorzugtes Merkmal der vorliegenden Erfindung ist, dass die Markersequenz die Proteineigenschaften des Terminationfaktors nicht beeinträchtigt. Ein aktives essentielles Translationsprodukt ist vorteilhaft für eine erfolgreiche Kultivierung des genetisch veränderten Organismus. Die Bestimmung der Funktionalität des mit einer Markersequenz versehenen essentiellen Translationsproduktes erfolgt über ein für die Funktion des essentiellen Translationsproduktes spezifisches Assay. Hierzu wird ein DNA-Fragment codierend für das essentielle Translationsprodukt und die Markersequenz über eine Expressions-PCR translatiert. Die Funktionalität wird anhand der Syntheserate des Produktes, an dessen Synthese das essentielle Translationsprodukt beteiligt ist, beurteilt. Die Syntheserate in Gegenwart des nativen essentiellen Translationsproduktes wird mit der Syntheserate in Gegenwart des modifizierten essentiellen Translationsproduktes verglichen und darüber die Funktionalität beurteilt. Die Funktionalität des essentiellen Translationsproduktes ist durch die Markersequenz nicht beeinträchtigt, wenn die Produktsyntheserate des markierten essentiellen Translationsproduktes 10%, bevorzugt 40 bis 60 %, insbesondere über 90% der Syntheserate des nativen essentiellen Translationsproduktes beträgt.

Das Lysat aus einem stabil transformierten erfindungsgemäßen Organismus enthält bis zu 100 Gew.-% (bezogen auf die Gesamtmenge an Translationsprodukt) das essentielle Translationsprodukt in Fusion mit der Markersequenz und ist allenfalls nur geringfügig (< 10 Gew.-%, sogar < 1 Gew.-%, bezogen auf die Gesamtmenge an Translationsprodukt) mit dem natürlichen essentiellen Translationsprodukt kontaminiert. Durch die Markersequenz ist das im Lysat unerwünschte essentielle Translationsprodukt leicht und effektiv aus dem Lysat entfernbar. Infolgedessen ist die Proteinbiosynthese synthetischer Proteine, in welche nicht-natürliche Aminosäuren eingebaut sind, schneller, mit höheren Ausbeuten und einer geringeren Anzahl von Nebenprodukten durchführbar.

Ein weiterer Vorteil der Erfindung ist, dass speziell nur eine unerwünschte Komponente aus dem Lysat entfernt werden kann. Es kann aber auch vorgesehen sein, dass mehrere verschiedene unerwünschte Translationsprodukte mit verschiedenen Markersequenzen, vorteilhafterweise jedoch mit der gleichen versehen sind, so dass mit einer Trennmethode alle unerwünschtenn Translationsprodukte entfernt werden können. Insofern kann die Verfahrensstufe a) für verschiedene Translationsprodukte ausgeführt werden, wobei die Markersequenz jeweils gleich oder verschieden sein kann.

### Ausführungsformen der Erfindung

Die Klonierung des Organismus kann durch in Fachkreisen bekannte Transformationsmethoden wie Mikroinjektion, Elektroporation oder durch chemisch vermittelte Aufnahmen der DNA erfolgen.

Die Isolierung des erfolgreich klonierten Organismus erfolgt anhand der Selektionssequenz nach in Fachkreisen bekannten Verfahren.

Die Kultivierung des Organismus kann im Batch-, Fed-Batch oder kontinuierlichen Verfahren erfolgen.

Ebenso kann die Proteinbiosynthese von synthetischen Proteinen enthaltend nicht-natürliche Aminosäuren im Batch-, Fed-Batch oder kontinuierlichen Verfahren erfolgen.

Die Lyse der Zellen erfolgt beispielsweise durch mechanische Einwirkung wie Hochdruckhomogenisation, durch Ultraschall oder durch Aufschluss in Kugelmühlen.

In einer besonderen Ausführungsform ist die Markersequenz ausgewählt aus der Gruppe "StrepTag-II, Polyhistidin, FLAG, Polyarginin, Polyaspartat, Polygluthamin, Polyphenylalanin, Polycystein, Myc, Gluthadion-S-Transferase, Protein A, Maltose bindendes Protein, Galactose bindendes Protein, Chloramphenicol-Acetyltransferase". Weitere Beispiele sind in den Patentansprüchen angegeben.

Die Markersequenz und das chromosomale Gen des essentiellen Translationsproduktes werden als Fusionsprotein exprimiert. In einer bevorzugten Ausführungsform ist die Markersequenz ein StrepTag-II, eine Peptidstruktur aus 8 Aminosäuren mit Affinität zu StrepTactin. So kann beispielsweise der exprimierte Terminationsfaktor RF1 am c-terminalen Ende das StrepTag-II aufweisen. Die Abtrennung des RF1-StrepTagII-Fusionsproteins erfolgt entsprechend an einer mit StrepTactin beladenen Affinitätsmatrix oder anderen SII-bindenden Matrices. Die Abtrennung kann auf der Basis säulenchromatographischer Verfahren, aber auch über Batchverfahren erfolgen. Es versteht sich, dass auch eine andere Markersequenz und ihr entsprechender Affinitätspartner anwendbar ist. Beispielhaft sei hier das PolyHis-Tag genannt. Ein PolyHis-Tag besteht in der Regel aus sechs aufeinanderfolgenden Histindinresten, kann in seiner Länge aber zwischen 4 bis 10 Resten varieren. In einer anderen bevorzugten Ausführungsform erfolgt die Isolierung der essentiellen Translationsprodukte über entsprechende Antikörper, Antikörperfragmente oder über Aptamere. Unter Umständen bewirkt die Affinität der Bindungspartner auch gleichzeitig eine Inhibition der Aktivität des essentiellen Translationsproduktes.

Hinsichtlich der Wahl der Methode zur Proteinabtrennung ist eine Methode auszuwählen, welche die Translationsaktivität des Lysates nicht negativ beeinflußt, d.h. wichtige Reaktionskomponenten des Translationssystems nicht abgetrennt.

Grundsätzlich kann der Organismus eine Eukaryont oder Prokariont sein. Vielfach hilfreich ist, wenn der Organismus zur Herstellung eines Lysates ein Prokaryont ist. Ergänzend wird auf die Patenansprüche verwiesen. Besonders geeignet ist das Translationssystem aus Escherichia coli.

Die Erfindung lehrt desweiteren ein Lysat nach Anspruch 6 und dessen Verwendung gemäß den Ansprüchen 7 und 8. In einer bevorzugten Ausführungsform weist das Lysat eine verminderte Aktivität des an der Termination beteiligten Faktors RF1 auf. Ein Beispiel für die Herstellung modifizierter synthetischer Proteine ist der Einbau von Biotinyllysin (Biocytin) mittels einer mit der Aminosäure aminoacylierten *amber*-Suppressor-tRNA. Bezüglich der Synthese und Aufreinigung biotinylierter oder anderer StrepTactin-bindender Proteine weist das System einen weiteren Vorteil auf: Da endogene biotinylierte Proteine während der Abtrennung von RF1-II ebenfalls abgetrennt werden, wird eine Kontamination von syntheischen Proteinen, die mit Hilfe von Strepavidin oder ähnlichen Matrices aufgereinigt werden, mit biotinylierten Proteinen aus dem Produktionsstamm vermieden. Das Lysat ermöglicht aber auch den effizienteren Einbau anderer funktioneller Gruppen in Proteine, besonders bevorzugt den Einbau von Fluorophoren, - oder den einer universell reaktiven Gruppe, über die andere Funktionen selektiv und ortsspezifisch gekoppelt werden können. Eine Verwendungsalternative ist auch der Einbau von natürlichen Aminosäuren, die beispielsweise isotopenmarkiert oder selenhaltig vorliegen können.

Die Beladung der *amber*-Suppressor-tRNA mit der unnatürlichen Aminosäure kann mittels der sogenannten chemischen Aminoacylierung erfolgen oder auch mit Hilfe von Enzymen, beispielsweise Synthetasen oder Ribozyme. Es ist auch möglich, enzymatische und verschiedene chemische Methoden miteinander zu kombinieren. Beispielsweise kann die tRNA erst chemisch oder enzymatisch mit Lysin, Cystein oder einer anderen Aminosäure, die eine reaktive Funktion in der Seitenkette enthält, aminoacyliert werden. An die entsprechende Aminoacyl-tRNA wird dann über die reaktive Funktion der Aminosäure eine interessante funktionelle Gruppe, beispielsweise ein Fluorophor, mit Hilfe gebräuchlicher chemischer Methoden gekoppelt. So kann beispielsweise die Sulfhydrylgruppe von Cystein über Maleimid modifiziert werden oder eine Aminogruppe über einen NHS-Ester. Die Aminoacylbindung der tRNA kann während der Modifikation stabilisiert sein, beispielsweise durch das Vorhandensein einer Schutzgruppe an der alpha-Aminogruppe.

Das System eignet sich für die Beantwortung und Lösung wissenschaftlicher Fragestellungen der Proteinforschung. Weiterhin eignet sich das System prinzipiell für ein Ribosomen-Display, da nach der Entfernung eines Terminationsfaktors das entsprechende Codon nicht abgelesen werden kann und dadurch der ribosomale Komplex aus mRNA, synthetischem Protein und Ribosom eine erhöhte Stabilität aufweist. Das System erlaubt auch eine definierte Einführung von Puromycin oder entsprechender Derivate an der Position des oben genannten Codons. Puromycin konkurriert normalerweise mit dem ternären Komplex oder Terminationsfaktoren und wird statistisch an das Ende der wachsenden Proteinkette angefügt. Die Generierung eines "ausgehungerten" Codons durch die Entfernung eines Terminationsfaktors ermöglicht den definierten Einbau von Puromycin an dieser Position. Auf diese Weise können an synthetische Proteine Funktionen angehängt werden, die an das Puromycin gekoppelt werden, beispielsweise DNA-Oligomere, Zucker oder andere Bausteine.

In einer anderen Ausführungsform kann das Lysat auch eine verminderte Aktivität eines anderen essentiellen Translationsproduktes aufweisen, beispielsweise eines aus der Gruppe der Phosphatasen, der Nukleasen, der Synthetasen oder Proteasen. Hierdurch kann die Herstellung solcher synthetischer Proteine verbessert werden, deren Synthese durch die Aktivität anderer essentieller Translationsprodukte als durch die Aktivität der Terminationsfaktoren limitiert wird.

Es ist auch möglich, mit Hilfe der dargelegten Methode bestimmte essentielle Translationsprodukte aus dem Lysat zu entfernen, die eine Beantwortung bestimmter wissenschaftlicher Fragestellungen stören, oder deren Entfernung eine Untersuchung bestimmter Fragestellungen erst ermöglicht.

Im Folgenden wird die Erfindung anhand von nicht limitierenden Beispielen näher erläutert.

### Beispiel 1: Konkurrenzverhalten von RF1 und amber-Suppressor-tRNA

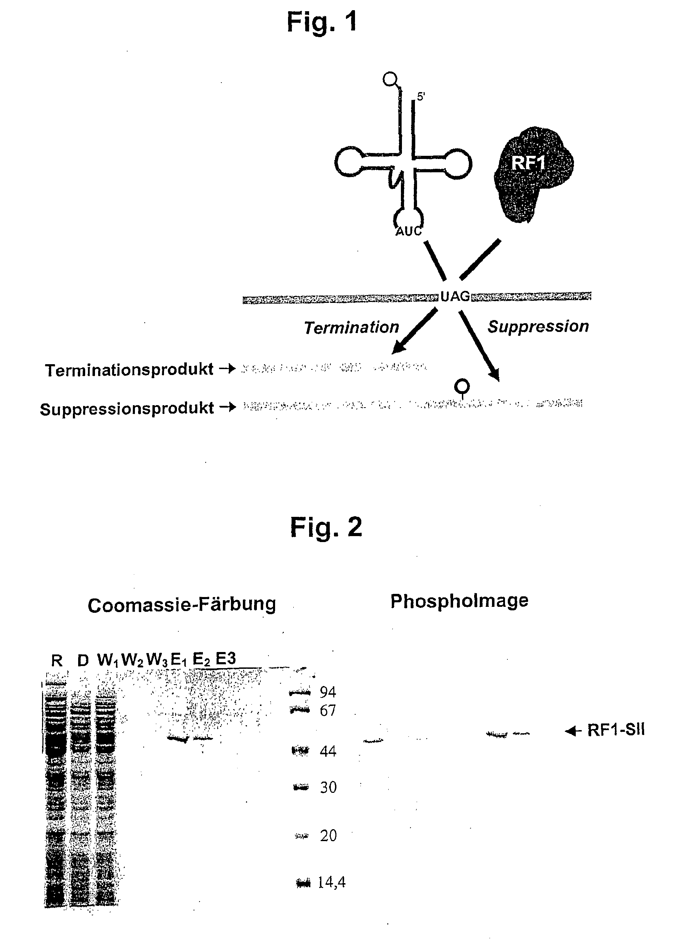

In der Figur 1 ist eine schematische Darstellung des Konkurrenzverhaltens von RF1 und einer amber-Suppressor-tRNA dargestellt. Je nachdem welches der beiden Moleküle mit dem Codon UAG paart, wird das Protein terminiert oder eine Aminosäure eingebaut und die Translation mit der Bildung des Suppressionsproduktes fortgeführt.

### Beispiel 2: Voruntersuchungen zur Funktionalität von RF1-SII: Expressions-PCR

Da eine Inaktivierung des Terminationsfaktors RF1 lethal für den Organismus wäre, wurde der Einfluß des angefügten StrepTags II auf die Aktivität von RF1 untersucht. Für diese Untersuchung wurde RF1 ausschließlich von Expressions-PCR-Produkten translatiert. Figur 2 zeigt die präparative Expression und Aufreinigung von RF1-SII. R entspricht der in vitro Translationsreaktion, D dem Durchlauf, W1, W2, W3 den Waschfraktionen und E1, E2, E3 den Elutionsfraktionen.

### Beispiel 3: Voruntersuchungen zur Funktionalität von RF1-SII: Amber-Suppressor-Assay

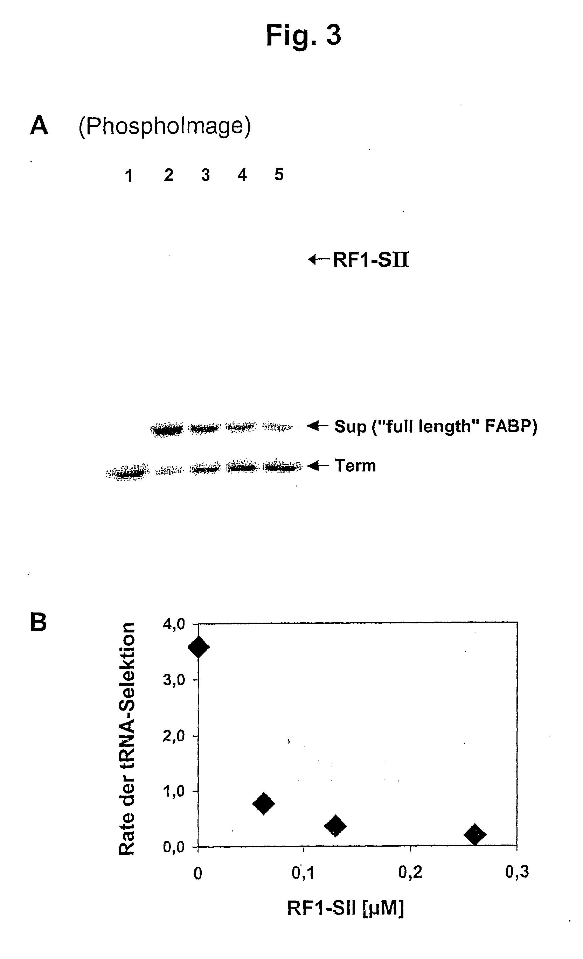

Figur 3 zeigt den Funktionstest von RF1-SII im amber-Suppressor-Assay. Die Ziffern 1 in Figur 3A bezeichnet die Durchführung des Assays in einem Ansatz ohne Zugabe von Suppressor-tRNA. Die Ziffern 2 bis 5 sind Ansätze mit Suppressor-tRNA (1 µM). Ansatz 2 enthält kein RF1-SII. Die Ansätze 3 bis 5 sind mit aufgereinigtem RF1-SII (3: 0,0625 µM, 4: 0,13 µM, 5: 0,26 µM) angereichert. Figur B zeigt die Rate der tRNA-Selektion in Abhängigkeit von der Zugabe von RF1-SII. Die "Rate der tRNA-Selektion" wird berechnet, indem anhand eines PhosphoImage die molaren Mengen an synthetischem Suppressions- und synthetischem Terminationsprodukt bestimmt werden und der Quotient aus den beiden Werten gebildet wird. Die Erhöhung des RF1-SII-Anteils im Ansatz führt zu einer vermehrten Produktion des Terminationsproduktes. Figur 3B zeigt die Rate der tRNA-Selektion in Abhängigkeit von der Mengen RF1-SII im Ansatz. Die tRNA-Selektionrate sinkt mit der Zugabe von RF1-SII von 3,5 auf unter 1 ab und kann durch Erhöhung des RF1-SII-Anteils weiter reduziert werden. Dies bestätigt, dass RF1-SII prinzipiell aktiv ist.

### Beispiel 4: Voruntersuchungen zur Funktionalität von RF1-SII: Aktivitätsvergleich mit nativem RF1

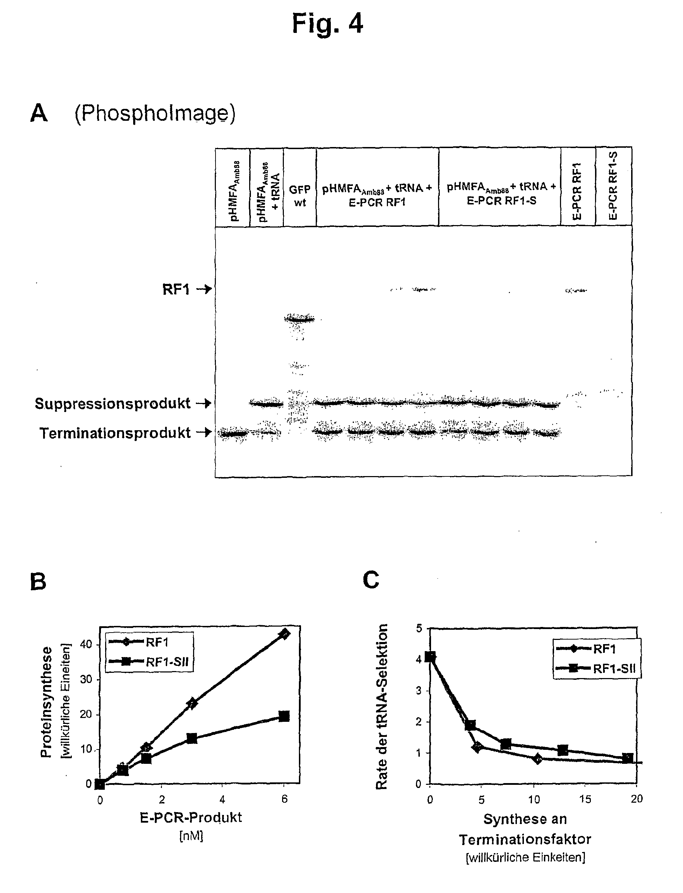

Figur 4 stellt den Vergleich der Funktionalität und Aktivität von getaggtem und nativem RF1 im Amber-Suppressions-Assay dar. RF1-SII zeigt gegenüber RF1 eine vergleichbare Aktivität. Figur 4B zeigt für RF1-SII in Abhängigkeit von der zugegebenen Menge an Matrize gegenüber RF1 eine geringere Syntheserate. Unter Berücksichtigung der Syntheseraten von RF1-SII und nativem RF1 wurden dann die Raten der tRNA-Selektion in Gegenwart beider Proteine bei der Expression des Reporterproteins FABPAmb88 aus dem PhosphoImage (Fig. 4A) bestimmt. Die für das Reporterprotein kodierende Matrize (pHMFAAmb88) enthält eine *amber-*Mutation an Aminosäureposition 88. Die Rate der tRNA-Selektion in Gegenwart von RF1-SII ist nahezu identisch mit der in Gegenwart von nativem RF1 (Diagramm 4C). Beide Proteine weisen somit eine vergleichbare Aktivität auf.

### Beispiel 5: Simulation der Entfernung von RF1-SII aus Lysaten

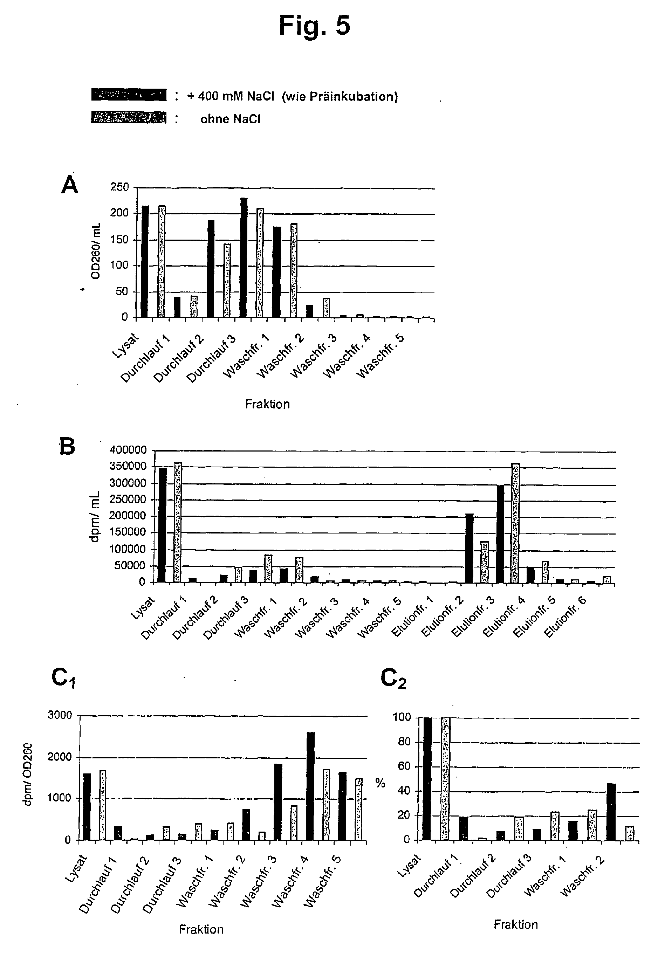

Zur Simulation der Entfernung von RF1-SII aus Lysaten wurde RF1-SII präparativ hergestellt und mit ¹⁴C-Leucin (100 dpm/pmol) markiert. Im Anschluß daran wurde das synthetisierte, aufgereinigte RF-SII einem S30-Lysat in einer Endkonzentration von 0,1 µM (in 1x TLM-Puffer, 215 A₂₆₀/ml)zugegeben. Die Abtrennung des RF1-SII erfolgt über eine StrepTactin-Säule, wobei insgesamt 500 µl Lysat (= ca. 110 A₂₆₀) auf 200 µl Säule in drei Schritten ä 166 µl aufgetragen wurden. Das Waschvolumen betrug je 200 µl. In Figur 5 ist das Elutionsverhalten von Lysatkomponenten und speziell von RF1-SII mit und ohne Zugabe von NaCl sowie der jeweilige Anteil von RF1-SII im Lysat dargestellt. Figur 5A zeigt das Elutionsverhalten von Lysatkomponenten. Aus Figur 5A ist zu entnehmen, dass die Lysatkomponenten größtenteils nicht oder nur unspezifisch an der Säule gebunden wurden. Unspezifisch gebundene Lysatkomponenten wurden durch Waschen leicht wieder eluiert (Waschfraktionen). Das verwendete Verfahren mindert somit nicht die Aktivität des Lysates durch Abtrennung erwünschter Lysatkomponenten. In Figur 5B ist das Elutionsverhalten von RF1-SII dargestellt und offenbart, dass RF1-SII spezifisch an der StrepTactin-Säule bindet und erst durch die Elutionslösung von der Säule eluiert wird (Elutionsfraktion, Figur 5B). In den Fraktionen des Durchlaufs sowie des Waschen ist RF1 nur geringfügig enthalten. Die Figuren C1 und C2 zeigen den Anteil von RF1-SII im Lysat in Abhängigkeit vom jeweiligen Abtrennungsschritt. Figur C1 enthält die Werte dpm RF1/ml im Verhältnis zu OD260/ml des Lysates. Der Anteil von RF1-SII (dpm/OD260) im reinen Lysat in Figur 5C1 ist in Figur 5C2 gleich 100% gesetzt, sodass Figur 5C2 den prozentualen Anteil von RF1-SII im Lysat darstellt. Figur 5C2 zeigt, dass RF1-SII nach den Abtrennungsschritten "Durchlauf" und Waschfraktion" in deutlich geringem Anteil im Lysat enthalten ist.

### Beispiel 6: genomische Struktur eines genetisch veränderten Organismus

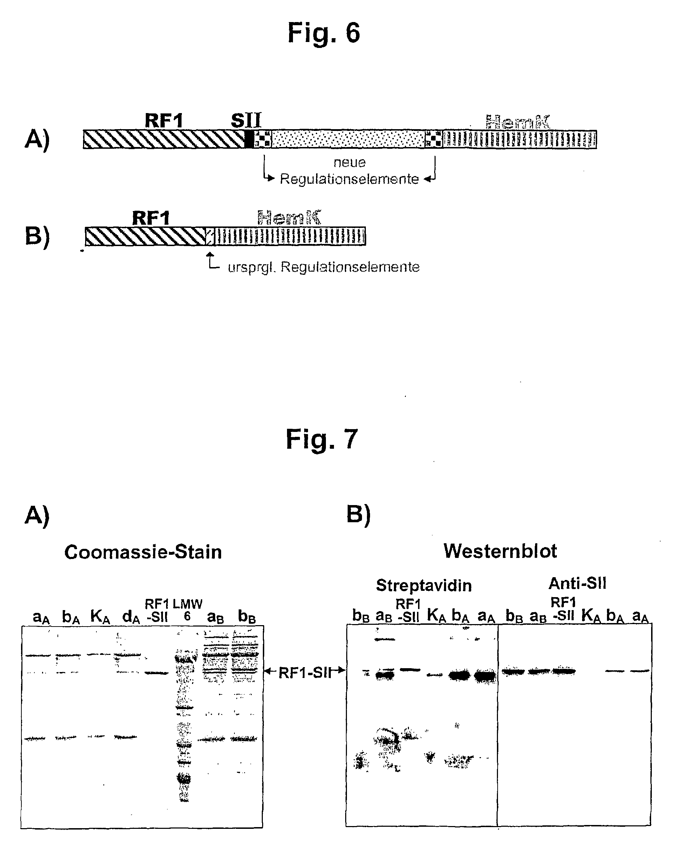

In der Figur 6 ist eine schematische Darstellung des chromosomalen Gens eines erfindungsgemäß ausgetauschten Proteins vor und nach der Klonierung dargestellt. Man erkennt die ursprüngliche genomische Situation (Figur 6B), welche aus dem RF1-Gen, einem Regulationselement und dem Gen für HemK besteht und die gewünschte genetische Situation (Figur 6A), in welcher an das Gen von RF1 die Markersequenz von StrepTag-II angehängt ist. Desweiteren enthält die gewünschte genetische Situation eine Selektionssequenz, hier eine Antibiotikaresistenz gegen Kanamycin sowie neue regulatorische Elemente. Ein erfindungsgemäßer Organismus ist bei der Deutschen Sammlung von Mikroorganismen und Zellkulturen GmbH gemäß dem Budapester Vertrag hinterlegt unter der Nummer DSM 15756 (E. coli/RF1-SII).

### Beispiel 7: Herstellung eines an RF1 defizienten Lysates

Die Kultivierung dreier E. coli /RF1-SII Klone (a,b,d) wurde in Schüttelkulturen vorgenommen. Die Kulturen wurden in der log-Phase geerntet und mit Hilfe von Ultraschall aufgeschlossen. Das RF1-SII haltige Lysat wurde in zwei Ansätze geteilt und RF1-SII durch unterschiedliche Methoden abgetrennt. Aus Ansatz A (in Figur 5 entsprechend Index A) wurde RF1-SII durch Affinitätschromatographie an einer StrepTactin-Säule (500 µl Lysat (= ca. 110 A₂₆₀) auf 200 µl Säule) abgetrennt. Der Ansatz B (in Figur 7 entsprechend Index B) wurde einer Präinkubation (400 mM NaCl) unterworfen und im Anschluß daran RF1-SII über eine StrepTag-Säule (500 µl Lysat (= ca. 125 A₂₆₀) auf 200 µl Säule) abgetrennt. Hiernach erfolgte Entsalzung über NAP 5. Die Ergebnisse sind der Figur 7 A und B zu entnehmen, welche den Nachweis von RF1-SII mittels SDS-Page und Westernblot im Elutionsvolumen zeigt. Figur 7A zeigt die Coomassieblaufärbung des Geles. Figur 7B zeigt die Detektion von RF1-SII mit Streptavidin-HRP oder Anti-SII (monoklonale Antikörper gegen StrepTag). Als Standard dient in vitro translatiertes und aufgereinigtes RF1-SII. LMW6 ist ein Molekulargewichtsmarker, K_{A} ein Lysat aus einem gentisch unveränderten E. Coli-Stamm, welches der Abtrennungsmethode des Ansatzes A unterworfen wurde. Die Ergebnisse zeigen, dass RF1-SII durch beide Methoden aus dem Lysat erfolgreich abgetrennt wurde.

### Beispiel 8: Expression von RF1-SII

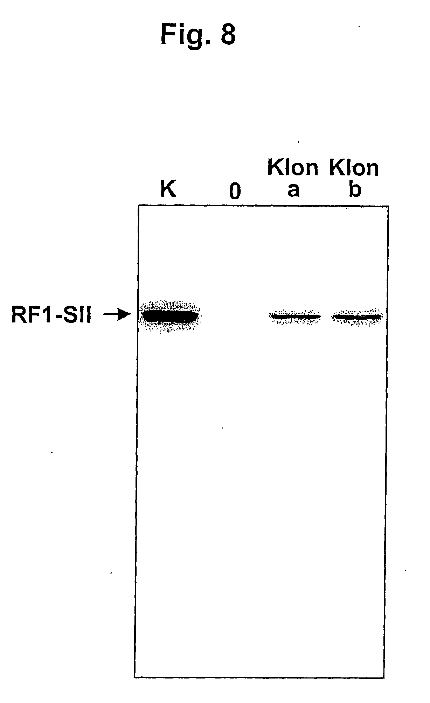

Es wurden zwei E. coli-Stämme mit dem unter Beispiel 6 angegebenen synthetischen DNA-Fragment (gewünschte genetische Situation) geklont. Mit Hilfe der Expressions-PCR wurden die Proteine RF1 und HemK aus der chromosomalen DNA (E. Coli K12) amplifiziert, kloniert und sequenziert. Über die PCR wurde auch die StrepTag-Sequenz (SII) an das Gen für RF1 angefügt sowie die neuen Regulationselemente für die Expression von HemK eingefügt. Beide Proteine wurden zellfrei translatiert, um ihre Exprimierbarkeit sowie im Falle von RF1 auch ihre Funktionalität zu überprüfen. Hierauf begann die Herstellung der Genkassette mit der gewünschten genomischen Situation für den chromosomalen Austausch. Es wurden drei PCR-Fragmente (mit den Genen für RF1-SII, für die Kanamycin-Resistenz sowie für HemK) hergestellt und miteinander ligiert. Die Ligation erfolgt unter Verwendung asymmetrischer Restriktionsschnittstellen in einer Eintopfreaktion, d.h. die drei Fragmente wurden in einem Schritt miteinander ligiert. Das entstehende DNA-Fragment mit der gewünschten genomischen Situation wurde geleluiert, in einen Vektor kloniert, sequenziert und mit Hilfe der PCR amplifiziert. Hierauf erfolgte die Transformation des PCR-generierten linearen Fragments in E. coli D10 mit Hilfe der Elektroporation. Die Kanamycin-Resistenz wurde für die Selektion auf Klone mit der gewünschten genomischen Situation verwendet. Hierzu wurden die Zellen auf Kanamycin-Platten ausgestrichen. Die vier positiven Klone wurden einer Gegenselektion in ampicillinhaltigem Medium unterzogen, um ausschließen zu können, dass das für die Amplifikation des Genfragmentes verwendete Plasmid, das eine Ampicillin-Resistenz trägt, transformiert wurde. Weiterhin wurde mit Hilfe der Kolonie-PCR das Vorhandensein des gewünschten Genfragmentes innerhalb des E. Coli-Chromosoms überprüft. Hierzu wurde ein Primer, der innerhalb der Kassette hybridisiert, mit einem Primer kombiniert, der im E. coli-Chromosom außerhalb der transformierten Kassette hybridisiert. Alle vier positiven Klone wiesen die gewünschte genomische Situation auf. Figur 8 zeigt die in vivo Expression von RF1-SII im Westernblot. Die Abtrennung von RF1-SII erfolgte über eine StrepTactin-Säule. Der Nachweis des Proteins wurde mit Anti-SII (monoklonaler Antikörper gegen StrepTag) durchgeführt. Die Figur 8 zeigt eine deutliche Expression von RF1-SII in den beiden Klonen a und b. Die Negativkontrolle "0" aus einem genetisch unverändertem Stamm zeigt keine Expression von RF1-SII. Die Probe "K" ist in vitro translatiertes RF1-SII und dient als Marker und Positivkontrolle.

### Beispiel 9: Einfluß der RF1-Abtrennung auf die Effizienz der Suppression im regenerierbaren System.

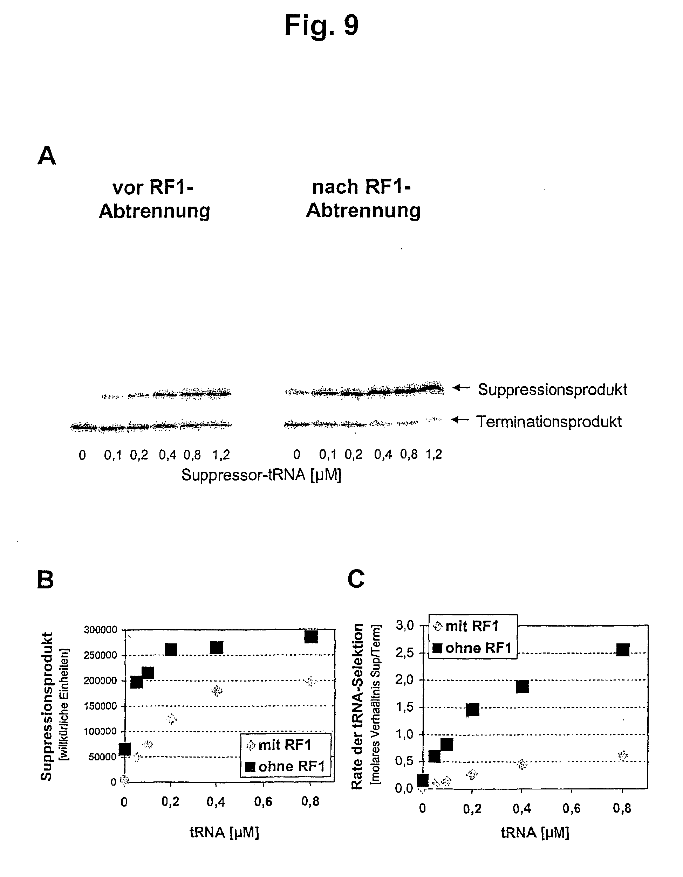

Die Figur 9 stellt das Ergebnis der in vitro Proteinbiosynthese mit einem erfindungsgemäßen Lysat und einem RF1 haltigen Lysat dar. Figur 9A zeigt das PhoshoImage eines SDS-Gels, welches die jeweiligen Anteile des Terminationsproduktes und des Suppressionsproduktes vor und nach der Abtrennung von RF1 in Abhängigkeit von der Menge an Suppressor-tRNA zeigt. In diesem Fall wurde eine enzymatisch aminoacylierbare tRNA verwendet. Bei einem Suppressor-tRNA-Anteil von 1,2 µM im RF1-defizienten Lysat sind nur noch geringe Mengen des Terminationsproduktes detektierbar. Durch Abtrennung von RF1 wird die Translation des Suppressionsproduktes erhöht (Figur 9B) und gleichzeitig das Verhältnis Suppressionsprodukt/Terminationsprodukt auf die Seite des Suppressionsproduktes verschoben (Figur 9C). Zudem wird die Syntheserate des Suppressionsproduktes durch höhere Zugabemengen der Suppressor-tRNA gesteigert. Im Ergebnis wird durch Abtrennung von RF1 aus einem Lysat die Syntheserate eines Suppressionsproduktes deutlich erhöht und somit die Ausbeute gesteigert.

### Beispiel 10: Einbau einer nicht-natürlichen Aminosäure

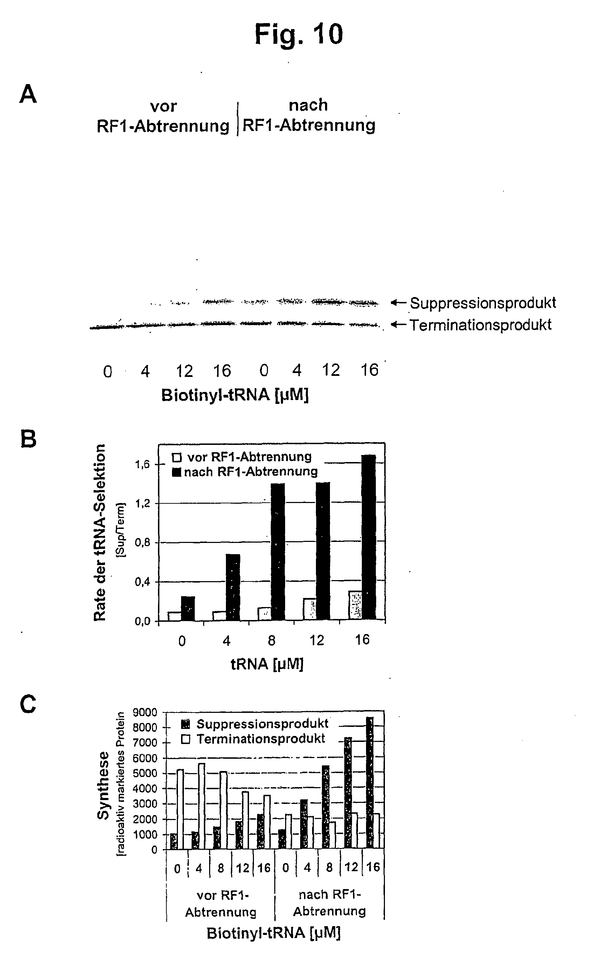

Die Figur 10 zeigt beispielhaft den gesteigerten Einbau von Biotinyllysin in Abwesenheit von RF1 in FABP (Figur 10A, PhosphoImage). Es wurde eine amber-Suppressor tRNA verwendet, die über chemische Methoden mit Biotinyllysin (Biocytin) beladen wurde. Die Markierung der translatierten Proteine mit ¹⁴C-Leucin bestätigt die höhere Syntheserate an biotinyliertem FABP in einem RF1-defizienten Lysat (Figur 10B und C).

### Beispiel 11: Einbau von Biocytin mit Hilfe einer mit chemischen Methoden beladenen amber-Suppressor-tRNA - Nachweis biotinylierter Proteine im Westernblot

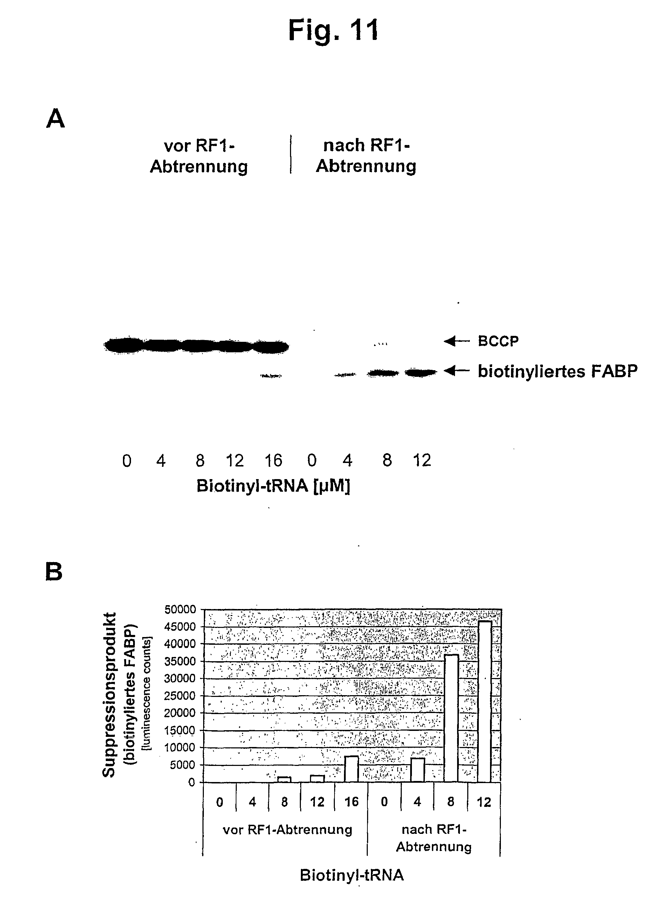

Fig. 11A zeigt den Westernblot, 11B die Quantifizierung des Westernblots über die Detektion von Chemilumineszenz. Es wurde ein monoklonaler Antikörper gegen StrepTag II verwendet, der mit HRP gekoppelt war. Der Westernblot zeigt eindeutig die stark erhöhte Synthese an synthetischem biotinyliertem Protein im Lysat nach RF1-Abtrennung. Weiterhin zeigt der Blot, daß durch die verwendete Methode zur Herstellung des RF1-defizienten Lysat auch endogene biotinylierte Proteine entfernt werden: Das in Lysaten aus *E. coli* relativ hochkonzentrierte endogene Protein BCCP wird nach RF1-Abtrennung kaum noch detektiert. Die Quantifizierung des Westernblots zeigt noch einmal die stark erhöhte Synthese an synthetischem modifiziertem Protein im RF1-defizienten Lysat und bestätigt die über Radioaktivität durchgeführte Quantifizierung aus Beispiel 10.

### Beispiel 12: Einbau von Biocytin in Abhängigkeit von der Reaktionszeit

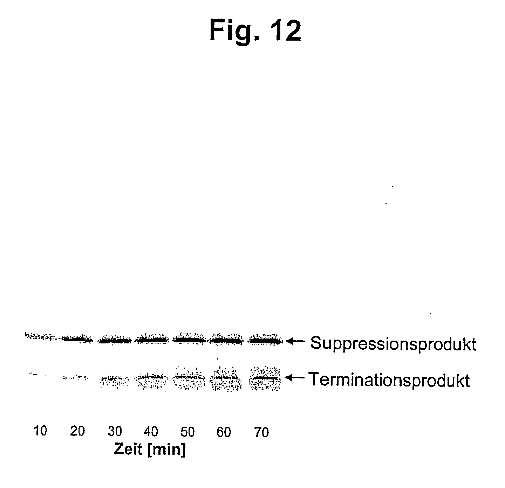

Mit längerer Reaktionszeit wird, wie Figur 12 zeigt, vermehrt Biotinyllysin (Biocytin) eingebaut. Infolgedessen erhöht sich der Anteil an Suppressionsprodukt an der Gesamtproduktmenge. Durch längere Reaktionszeiten wird die Ausbeute des biotinylierten Suppressionsproduktes gesteigert.

## Patentansprüche

1. Verfahren zur zellfreien Proteinbiosynthese umfassend ein Verfahren zur Herstellung eines Zelllysates mit folgenden Verfahrensschritten:
a) Austausch einer für ein essentielles, jedoch die Ausbeute einer zellfreien Proteinbiosynthese reduzierendes Translationsprodukt, ausgewählt aus Terminationsfaktor 1 (RF1), Terminationsfaktor 2 (RF2) und eukaryontischer Terminationsfaktor (eRF), codierenden genomischen Sequenz in einem Organismus gegen eine unter einem geeigneten regulatorischen Element stehende fremde DNA, wobei die fremde DNA für das essentielle Translationsprodukt, jedoch zusätzlich enthaltend eine Markersequenz co-diert,
b) Kultivieren des gemäß a) transformierten Organismus,
c) Lyse des Organismus aus der Kultur nach b) und
d) Abtrennung des essentiellen Translationsproduktes aus dem in c) erhalte-nen Lysat mittels eines für die Markersequenz selektiven Trennverfahrens.

2. Verfahren nach Anspruch 1, **dadurch gekennzeichnet, dass** die Markersequenz ausgewählt ist aus der Gruppe "StrepTag-II, Polyhistidin, FLAG, Polyarginin, Polyaspartat, Polyglutamin, Polyphenylalanin, Polycystein, Myc, Gluthadion-S-Transferase, Protein A, Maltose bindendes Protein, Galactose bindendes Protein, Chloramphenicol-Acetyltransferase, Protein G, Calmodulin, Calmodulin-bindendes Peptid, HAT (= natural histidine affinity tag), SBP (= Streptavidin-bindendes Peptid), Chitin-bindende Domäne, Thioredoxin, ß-Galaktosidase, S-Peptid (Reste 1-20 der RNase A), Avidin, Streptavidin, StrepTag-I, Dihydrofolat-Reduktase, *lac*-Repressor, Cyclomaltodextrin-Glucanotransferase, Cellulose-bindende Domäne, Btag, NanoTag".

3. Verfahren nach einem der Ansprüche 1 oder 2, wobei die Markersequenz und die genomische Sequenz als Fusionsprotein exprimiert werden und wobei die translatierte Markersequenz die Aktivität des essentiellen Translationsproduktes im Organismus nicht beeinträchtigt.

4. Verfahren nach einem der Ansprüche 1 bis 3, wobei das Trennverfahren eine Affinitätschromatographie oder ein Antikörper-Assay ist.

5. Verfahren nach Anspruch 1 bis 4, **dadurch gekennzeichnet, dass** der Organismus ein Prokaryont oder Eukaryont ist, insbesondere ausgewählt ist aus der Gruppe bestehend aus "Enterobacteriales (z.B. *Escherichia* spec., E.coli), Lactobacillales (z.B. *Lactococcus* spec., *Streptococcus* spec.), Actinomycetales (z.B. *Streptomyces* spec., *Corynebacterium* spec.), *Pseudomonas* spec., *Caulobacter* spec., *Clostridium* spec., *Bacillus* spec., *Thermotoga* spec., *Micrococcus* spec., *Thermus* spec.".

6. Verfahren nach einem der Ansprüche 1 bis 5, wobei das Lysat eine verminderte Menge des essentiellen Translationsproduktes aufweist.

7. Verfahren nach Anspruch 1, wobei mittels amber-Suppressor-tRNAs natürliche und/oder nicht-natürliche Aminosäuren, bevorzugt Biotinyllysin, fluoreszierende Aminosäuren und/oder Phenylalanin, eingebaut werden.

## Claims

1. A method for the cell-free protein synthesis comprising a method for preparing a cell lysate comprising the following steps:
a) a genomic sequence, which encodes an essential translation product that however reduces the yield of a cell-free protein biosynthesis and is selected from termination factor 1 (RF1), termination factor 2 (RF2), and eukaryotic termination factor (eRF), in an organism is replaced by a foreign DNA located under a suitable regulatory element, said foreign DNA encoding the essential translation product, however additionally containing a marker sequence,
b) the transformed organism according to step a) is cultivated;
c) the organism from the culture obtained in step b) is lysed; and
d) the essential translation product is separated from the lysate obtained in step c) by means of a separation process that is selective for the marker sequence.

2. The method according to claim 1, **characterized in that** the marker sequence is selected from the group consisting of "streptag II, polyhistidine, FLAG, polyarginine, polyaspartate, polyglutamine, polyphenylalanine, polycysteine, Myc, gluthatione S-transferase, protein A, maltose-binding protein, galactose-binding protein, chloramphenicol acetyltransferase, protein G, calmodulin, calmodulin-binding peptide, HAS (= natural histidine affinity tag), SBP (= streptavidin-binding peptide), chitin-binding domain, thioredoxin, ß-galactosidase, S-peptide (residues 1-20 of RNase A), avidin, streptavidin, StrepTag-I, dihydrofolate reductase, lac repressor, cyclomaltodextrin glucanotransferase, cellulose-binding domain, BTAG, nanotag".

3. The method according to one of claims 1 or 2, wherein the marker sequence and the genomic sequence are expressed as a fusion protein, and wherein the translated marker sequence does not affect the activity of the essential translation product in the organism.

4. The method according to one of claims 1 to 3, wherein the separation step is an affinity chromatography or an antibody assay.

5. The method according to claim 1 to 4, **characterized in that** the organism is a prokaryote or eukaryote, in particular selected from the group consisting of "enterobacteriales (e.g. *Escherichia* spec., E.coli), lactobacillales (e.g. *Lactococcus* spec., *Streptococcus* spec.), actinomycetes (e.g. *Streptomyces* spec., *Corynebacterium* spec.), *Pseudomonas* spec., *Caulobacter* spec., *Clostridium* spec., *Bacillus* spec., *Thermotoga* spec., *Micrococcus* spec., *Thermus* spec."

6. The method according to one of claims 1 to 5, wherein the lysate has a reduced quantity of the essential translation product.

7. The method according to claim 1, wherein by means of amber suppressor tRNAs natural and/or non-natural amino acids, preferably biotinyl lysine, fluorescent amino acids and/or phenyl alanine are incorporated.

## Revendications

1. Procédé pour la biosynthèse de protéines sans cellules comprenant un procédé préparatif pour un lysat cellulaire comprenant les étapes suivantes:
a) échanger une séquence génomique codant pour un produit de traduction essentiel, qui cependant réduit le rendement de la biosynthèse de protéines sans cellules et est choisi à partir de facteur de terminaison 1 (RF1), facteur de terminaison 2 (RF2), et facteur de terminaison eucaryote (eRF), dans un organisme par un ADN étranger situé sous un élément régulateur approprié, l'ADN étranger codant pour le produit de traduction essentiel, cependant également contenant une séquence de marqueur,
b) cultiver l'organisme transformé selon a),
c) lyser l'organisme de la culture selon b), et
d) séparer le produit de traduction essentiel du lysat obtenu dans c) par un procédé de séparation étant sélectif pour la séquence de marqueur.

2. Procédé selon la revendication 1, **caractérisé en ce que** la séquence de marqueur est choisi à partir du groupe «étiquette strep II, polyhistidine, étiquette FLAG, polyarginine, polyaspartate, polyglutamine, polyphénylalanine, polycystéine, Myc, gluthation S-transférase, protéine A, protéine liant le maltose, protéine liant le galactose, chloramphénicol acétyltransférase, protéine G, calmoduline, peptide liant la calmoduline, HAS (= étiquette d'affinité histidine naturelle), SBP (= peptide liant la streptavidine), domaine liant la chitine, thiorédoxine, β-galactosidase, S-peptide (résidus 1-20 de RNase A), avidine, streptavidine, étiquette strep I, dihydrofolate réductase, lac répresseur, cyclomaltodextrine glucanotransférase, domaine liant la cellulose, BTAG, nanotag».

3. Procédé selon une des revendications 1 ou 2, dans lequel la séquence de marqueur et la séquence génomique sont exprimées comme protéine de fusion, et dans lequel la séquence de marqueur traduite ne dégrade pas l'activité du produit de traduction essentiel dans l'organisme.

4. Procédé selon une des revendications 1 à 3, dans lequel le procédé de séparation est une chromatographie d'affinité ou une analyse d'anticorps.

5. Procédé selon la revendication 1 à 4, **caractérisé en ce que** l'organisme est un procaryote ou eucaryote, en particulier choisi à partir du groupe consistant en «enterobacteriales (p.ex. espèce *Escherichia,* E.coli), lactobacillales (p.ex. espèce *Lactococcus,* espèce *Streptococcus*), actinomycetes (p.ex. espèce *Streptomyces,* espèce *Corynebacterium*), espèce *Pseudomonas*, espèce *Caulobacter*, espèce *Clostridium,* espèce *Bacillus,* espèce *Thermotoga,* espèce *Micrococcus,* espèce *Thermus*».

6. Procédé selon une des revendications 1 à 5, dans lequel le lysat a une quantité réduite du produit de traduction essentiel.

7. Procédé selon la revendication 1, dans lequel au moyen d'ARNt suppresseurs ambres, des acides aminés naturels et/ou non naturels, de préférence la biotinyllysine, des acides aminés fluorescents et/ou la phénylalanine sont incorporés.
